# EUROPEAN PATENT APPLICATION

(11) **EP 4 597 506 A1**
(43) Date of publication of application: **06.08.2025**
(21) Application number: 25150387.6
(22) Date of filing: 07.01.2025
(51) Int. Cl.: G16H 10/40, G16H 40/63

(54) **METHOD AND APPARATUS FOR DISPLAYING EXPERIMENTAL STATE OF BIOLOGICAL SAMPLE, AND SYSTEM FOR ANALYZING BIOLOGICAL SAMPLE**

(30) Priority: 31.01.2024 CN 202410144628
(71) Applicant: Shenzhen New Industries Biomedical Engineering Co., Ltd., Shenzhen, Guangdong 518122 (CN)
(72) Inventor: YIN, Li, Guangdong, 518122 (CN); HUANG, Ren, Guangdong, 518122 (CN); XIAO, Hao, Guangdong, 518122 (CN); TANG, Junhui, Guangdong, 518122 (CN); HU, Ying, Guangdong, 518122 (CN)
(74) Representative: Zacco GmbH

(57) **Abstract**

The present disclosure relates to a method and apparatus for displaying an experimental state of a biological sample, and a system for analyzing a biological sample, and relates to the technical field of vitro diagnosis. The method includes: in a case that an experimental state search instruction of a target sample is received, obtaining currently-executed step data of the target sample; determining experimental state data of the target sample according to preset experimental step data and the currently-executed step data; displaying the experimental state data on a first display area; and in a case that a tracking display instruction is received, obtaining a display sub-content corresponding to the experimental state data, generating symbol information corresponding to the target sample in the display sub-content, and displaying the display sub-content and the symbol information on a second display area.

## Description

### Cross-Reference to Related Application

This application claims priority to Chinese Patent Application No. 202410144628.2, filed to the China National Intellectual Property Administration on January, 31, 2024 and entitled "Method and Apparatus for Displaying Experimental State of Biological Sample, and System for Analyzing Biological Sample", the disclosure of which is hereby incorporated by reference in its entirety.

### Technical Field

The disclosure relates to the technical field of vitro diagnosis, in particular to a method and apparatus for displaying an experimental state of a biological sample, and a system for analyzing a biological sample.

### Background

In the field of in vitro diagnosis, most of existing sample analyzers including a biochemical analyzer, a immunoassay analyzer, a nucleic acid test analyzer, etc. have functions such as sample registration and sample result view. An experiment with the sample analyzer involves numerous steps. For example, the nucleic acid test analyzer integrates nucleic acid extraction, a polymerase chain reaction (PCR) system construction, nucleic acid amplification and testing, etc. A PCR experiment includes sample transfer, nucleic acid extraction, PCR system construction, and nucleic acid amplification and testing, each of which may be time-consuming. Hence, it is generally time-consuming from sample loading to result output and it even takes a couple of hours to obtain experimental results in a case of numerous samples during consecutive experiments. In a nutshell, it is a lengthy process from the sample loading to the result output and involves numerous sample processing steps. In view of that, it is essential to view and track a real-time state of a loaded sample, for which a better solution has not been provided yet at present.

### Summary

Embodiments of the present disclosure provide a method and apparatus for displaying an experimental state of a biological sample, and a system for analyzing a biological sample, which can at least display an experimental state data of a target sample in real time, and further display a display sub-content and symbol information corresponding to the experimental state.

According to one aspect of the present disclosure, a method for displaying an experimental state of a biological sample is provided. The method includes: in a case that an experimental state search instruction of a target sample is received, obtaining currently-executed step data of the target sample; determining experimental state data of the target sample according to preset experimental step data and the currently-executed step data; displaying the experimental state data on a first display area; and in a case that a tracking display instruction is received, obtaining a display sub-content corresponding to the experimental state data, generating symbol information corresponding to the target sample in the display sub-content, and displaying the display sub-content and the symbol information on a second display area.

According to one aspect of the present disclosure, an apparatus for displaying an experimental state of a biological sample is further provided. The apparatus includes: an obtainment component configured to obtain, in a case that an experimental state search instruction of a target sample is received, currently-executed step data of the target sample; a data component configured to determine experimental state data of the target sample according to preset experimental step data and the currently-executed step data; a first display component configured to display the experimental state data on a first display area; and a second display component configured to, in a case that a tracking display instruction is received, obtain a display sub-content corresponding to the experimental state data, generate symbol information corresponding to the target sample in the display sub-content, and display the display sub-content and the symbol information on a second display area.

According to another aspect of the present disclosure, a system for analyzing a biological sample is further provided. The system includes: an experimental platform and a control platform; where the experimental platform is in communication connection to the control platform, the control platform includes an experiment control component and an information processing component, and the information processing component is configured to execute the method described above.

According to another aspect of the present disclosure, a system for analyzing a biological sample is further provided. The system includes: a test apparatus configured to obtain a sample test result by testing a sample liquid to be tested; a display; and a controller; where in a case that an experimental state search instruction of a target sample is detected, the controller is configured to control the display to display a first display area, and the first display area is configured to display experimental state data of the target sample; and in a case that a display search tracking operation of the target sample is detected, the controller is configured to obtain a display sub-content corresponding to the experimental state data, generate symbol information corresponding to the target sample in the display sub-content, and control the display to display a second display area, and the second display area is configured to display the display sub-content and the symbol information corresponding to the experimental state data of the target sample.

The embodiments of the present disclosure have the following beneficial effects:
According to the embodiments of the present disclosure, in the case that the experimental state search instruction of the target sample is received, the currently-executed step data of the target sample are obtained. The experimental state data of the target sample are determined according to the preset experimental step data and the currently-executed step data. The experimental state data are displayed on the first display area. In the case that the tracking display instruction is received, the display sub-content corresponding to the experimental state data are obtained, the symbol information corresponding to the target sample in the display sub-content is generated, and the display sub-content and the symbol information are displayed on the second display area. According to the present disclosure, real-time display and tracking display of the experimental state of the target sample can be implemented, and convenience is provided for searching for the experimental state of the biological sample.

Details of one or more embodiments of the present disclosure are set forth in the following accompanying drawings and descriptions to make other features, objectives and advantages of the present disclosure more clear and comprehensible.

### Brief Description of the Drawings

In order to more clearly describe technical solutions in the embodiments of the present disclosure or in the related art, a brief introduction to the accompanying drawings required for the description of the embodiments or the related art will be provided below. Apparently, the accompanying drawings in the following descriptions merely show some embodiments of the present disclosure, and a person of ordinary skill in the art can still derive other embodiments from the accompanying drawings without creative efforts.
Fig. 1 is a flowchart of a method for displaying an experimental state of a biological sample according to some embodiments of the present disclosure;
Fig. 2 is a flowchart of searching for and tracking a real-time state of a sample according to some embodiments of the present disclosure;
Fig. 3 is an entire architecture diagram of a system for analyzing a biological sample according to some embodiments of the present disclosure;
Fig. 4 is a schematic structural diagram of an electronic device according to some embodiments of the present disclosure;
Fig. 5 is an image of a sample real-time state search interface according to some embodiments of the present disclosure;
Fig. 6 is an image I of a sample tracking display interface according to some embodiments of the present disclosure;
Fig. 7 is an image II of the sample tracking display interface according to some embodiments of the present disclosure; and
Fig. 8 is an image III of the sample tracking display interface according to some embodiments of the present disclosure.

### Detailed Description of the Embodiments

The embodiments of the present disclosure will be described below in more detail with reference to the accompanying drawings. Although some embodiments of the present disclosure are shown in the accompanying drawings, it should be noted that the present disclosure can be implemented through various forms, and should not be constructed to be limited to the embodiments expounded herein. On the contrary, these embodiments are provided for more thorough and complete understanding of the present disclosure. It should be noted that the accompanying drawings and the embodiments of the present disclosure are merely for illustration rather than limitation to the protection scope of the present disclosure.

In an actual application scenario, hospitals/doctors have strict requirements and control over time of result output of a sample of a patient. The hospitals/doctors need to know approximate time required for result output, and even need to know a current processing stage of the sample and may decide to adjust an experimental sequence of the sample or change an instrument for an experiment according to the current experimental stage of the sample. Thus, it is quite necessary to provide a system for searching for and tracking a real-time state of the sample in instrument software. In related art, most of a biochemical analyzer, a immunoassay analyzer, a nucleic acid test analyzer, etc. on the market have functions such as sample registration and sample result view. However, a real-time state search and tracking function during an experiment process after the sample is loaded is not provided.

In view of that, the present disclosure provides a method and apparatus for displaying an experimental state of a biological sample, and a system for analyzing a biological sample, which can search for and track the real-time state of the experimental state of the biological sample. According to the present disclosure, a sample real-time state search interface is designed to search for the real-time state and estimated remaining time of the sample, and the sample is displayed and tracked through a sample registration interface and a result interface and by drawing a key part diagram corresponding to a mechanical structure of an instrument. Moreover, by drawing the key part diagrams corresponding to the mechanical structure of the instrument, a mechanical part where the sample is currently processed can be further tracked, and a subsequent processing position of the sample can be tracked in real time.

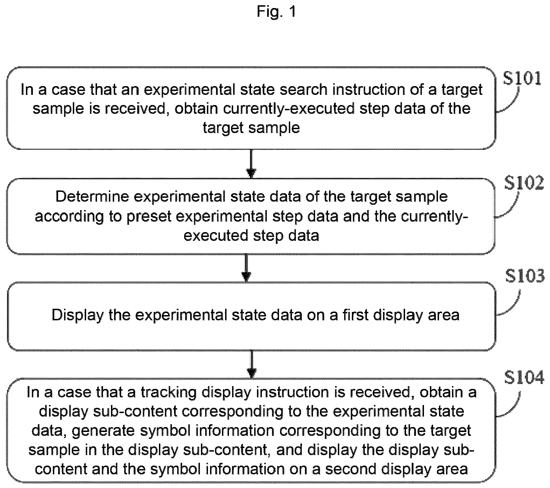

The embodiments of the present disclosure provide a method for displaying an experimental state of a biological sample. The method may be applied to a nucleic acid test analyzer and other sample analyzers in the field of vitro diagnosis, such as a biochemical analyzer and a immunoassay analyzer. Fig. 1 is a flowchart of a method for displaying an experimental state of a biological sample according to some embodiments of the present disclosure. Steps of the method involved in Fig. 1 will be described below.

Step 101, in a case that an experimental state search instruction of a target sample is received, currently-executed step data of the target sample are obtained.

In some embodiments of the present disclosure, the biological sample includes, but is not limited to, a sample, a control material and a calibrator. The control material refers to a reference material configured to calibrate performance of the instrument, verify accuracy of a test method and monitor stability of an experimental result. The control material usually includes a material to be tested of a known concentration or a matrix component in a simulated biological sample. The calibrator refers to a reference material configured to calibrate a laboratory testing device and determine accuracy of a measurement system. The sample refers to a raw material obtained from an organism (including a human, an animal, a plant and a microorganism) for a scientific research or diagnostic purpose. The target sample refers to one or several specified biological samples. For example, the target sample may be a urine sample of someone.

It should be noted that common types of biological samples include, but are not limited to: a body fluid sample: blood (whole blood, plasma or serum), urine, saliva, bile, cerebrospinal fluid, semen, cervical secretions and tears; a tissue sample: living tissue sections (skin, muscle, fat, nerves, etc.), tumor tissues removed through surgery, and normal tissue control samples; a cell sample: white blood cells, red blood cells, tissue culture cells, primary cells; a microbial sample: bacteria, viruses, fungi and protozoa; a sample including genetic materials (the genetic materials include DeoxyriboNucleic acid (DNA) and Ribonucleic acid (RNA)); and other biological samples including hair, nails, teeth, feces, exhaled gas, etc.

The experimental state search instruction is configured to search for an experimental stage of the biological sample. The experimental state search instruction may be transmitted by a user through human-computer interaction. In the case that the experimental state search instruction of the target sample is received, the currently-executed step data of the target sample are obtained. The currently-executed step data are data fed back by an instrument executing a biological sample experiment, and are configured to describe an executed state of a current step. For example, the currently-executed step data may be configured to indicate that uncapping is in process. It should be noted that data fed back by the instrument every time the instrument executes an experimental step may be stored. In the case that the experimental state search instruction of the target sample is received, data fed back on a latest executed experimental step corresponding to the target sample may be obtained from stored data, so as to obtain the currently-executed step data. In addition, the currently-executed step data may be obtained by directly obtaining the data fed back on the latest experimental step executed by the instrument. In this step, an obtainment operation of the currently-executed step data may be started every time the experimental state search instruction of the target sample is received, so as to provide data support for displaying the experimental state of the biological sample in real time.

S102, experimental state data of the target sample are determined according to preset experimental step data and the currently-executed step data.

In some embodiments of the present disclosure, the preset experimental step data are configured to describe experimental steps included in different experimental states, and may be set according to actual demand, which is not specifically limited in the embodiments of the present disclosure. After the currently-executed step data are determined, experimental state data to which the currently-executed step data belong can be determined by searching for the preset experimental step data, that is, the experimental state data of the target sample can be determined. The experimental state data are configured to describe a current progress of the experiment.

It should be noted that the currently-executed step data are configured to describe the experimental steps through codes or other agreed forms of data, which generally include one or more communication instructions between a controller and an experimental executor. Each communication instruction includes a request and a response. In general, the controller transmits a request command to request execution of a specific mechanical motion, and the experimental executor receives the request command, completes the specific mechanical motion according to a content of the request command, and then responds to the controller with a promise command. The experimental state data are configured to describe the experimental state with text and may be displayed to the user through a display apparatus. Thus, in this step, according to the preset experimental step data, interactive data between machines, that is, the currently-executed step data, may be converted into data understandable by human beings, that is, the experimental state data.

S103, the experimental state data are displayed on a first display area.

In some embodiments of the present disclosure, the first display area may be a complete display interface or a designated area in the display interface. The experimental state data are displayed on the first display area, such that the user can advantageously obtain the experimental state data in time.

S104, in a case that a tracking display instruction is received, a display sub-content corresponding to the experimental state data is obtained, symbol information corresponding to the target sample in the display sub-content is generated, and the display sub-content and the symbol information are displayed on a second display area.

In some embodiments of the present disclosure, the tracking display instruction is configured to further search for the display sub-content corresponding to the experimental state data. The display sub-content is configured to describe detailed information of biological samples in the experimental state. Thus, through the tracking display instruction, the user can further understand a specific situation of a current experimental state.

It should be noted that the user may transmit the tracking display instruction based on the first display area. For example, a tracking display button may be displayed in the first display area, and the user may transmit the tracking display instruction by clicking the tracking display button.

The symbol information is configured to symbolize a position of the target sample in the display sub-content. For example, the symbol information may be configured to symbolize and display, in a structural diagram of the instrument, a specific position of the sample in a structure of the instrument. The symbol information may be further configured to highlight the target sample in a sample list. A form of the symbol information can be set according to actual demand. For example, in some embodiments of the present disclosure, the step that symbol information corresponding to the target sample in the display sub-content is generated includes: one or more combinations of data corresponding to the target sample are generated as follows: shape data, color data and text data. The shape data may include, but are not limited to: a triangle, a circle, an underline, a curve, and a star. The color data may include, but are not limited to: red, orange and bold display. The text data may be selected according to actual demand.

For example, in some embodiments of the present disclosure, when a symbol is added to the sample, a red star may be used as the symbol. Any other visual effect of a software interface, such as a background color, other shapes/icons and bold font, may further be used as the symbol. However, core functions including the real-time state search of the sample and sample flow tracking are unchanged.

The second display area may be another complete display interface different from the first display area or a different designated area in the same display interface as the first display area.

In some embodiments of the present disclosure, the display sub-content and the symbol information are displayed on the second display area, such that the user can advantageously know the experimental state of the target sample.

According to the embodiments of the present disclosure, in the case that the experimental state search instruction of the target sample is received, the currently-executed step data of the target sample are obtained. The experimental state data of the target sample are determined according to the preset experimental step data and the currently-executed step data. The experimental state data are displayed on the first display area. In the case that the tracking display instruction is received, the display sub-content corresponding to the experimental state data are obtained, the symbol information corresponding to the target sample in the display sub-content is generated, and the display sub-content and the symbol information are displayed on the second display area. According to the present disclosure, real-time display and tracking display of the experimental state of the target sample can be implemented, and convenience is provided for searching for the experimental state of the biological sample.

In some embodiments of the present disclosure, the preset experimental step data include a correspondence relationship between the currently-executed step data and the experimental state data; the step that experimental state data of the target sample are determined according to preset experimental step data and the currently-executed step data includes: according to the correspondence relationship, experimental state data corresponding to the currently-executed step data are determined. The experimental state data include pre-experimental state data, in-experimental state data and post-experimental state data, a pre-experimental state includes at least one or more of the following states: registered and applied, an in-experimental state includes at least one or more of the following states: in-progress uncapping, in-progress sample adding, in-progress reagent adding, in-progress reacting and in-progress testing, and a post-experimental state includes at least one or more of the following states: result output completion and audit completion. The experimental state data corresponding to the currently-executed step data are taken as the experimental state data of the target sample.

In some embodiments of the present disclosure, the preset experimental step data are configured to determine experimental steps to be executed in the biological sample experiment. These experimental steps may be executed one by one by the instrument that executes the biological sample experiment. Data related to an experimental step being executed are taken as the currently-executed step data. The preset experimental step data include experimental state data to which experimental steps belong, while the currently-executed step data are relevant data of an experimental step. That is, the preset experimental step data include the correspondence relationship between the currently-executed step data and the experimental state data.

After the currently-executed step data are obtained, the preset experimental step data are searched for the correspondence relationship between the currently-executed step data and the experimental state data, and the experimental state data corresponding to the currently-executed step data can be obtained.

In order to facilitate understanding the current experimental stage by the user, the experimental state data include the pre-experimental state data, the in-experimental state data and the post-experimental state data. The pre-experimental state indicates that the biological sample experiment is in a preparation stage. The in-experimental state indicates that the biological sample experiment is in progress. The post-experimental state indicates that the biological sample experiment is completed. In order to facilitate further understanding details of the experimental state by the user, the pre-experimental state includes at least one or more of the following states: registered and applied, the in-experimental state includes at least one or more of the following states: uncapped, in-progress sample adding, in-progress reagent adding, in-progress reacting and in-progress testing, and the post-experimental state includes at least one or more of the following states: result output completion and audit completion.

It should be noted that the pre-experimental state includes at least specific states such as registered or applied, and the applied state indicates completion of a system application item of a laboratory information system (LIS). The in-experimental state includes at least specific states such as the in-progress uncapping, the in-progress sample adding, the in-progress reagent adding, the in-progress reacting and the in-progress testing. The post-experimental state includes at least specific states such as the result output completion and the audit completion. Specifically, specific states of the experimental stages may be determined according to the actual demand, which is not specifically limited by the embodiments of the present disclosure. For example, the in-experimental state may further include a specific state such as in-progress extraction.

The experimental state data corresponding to the currently-executed step data are taken as the experimental state data of the target sample, so as to be displayed to the user and make the user understand a current experimental progress.

In some embodiments of the present disclosure, in a case that the experimental state data indicate the in-experimental state data, the method further includes: total preset experimental time of the target sample is determined according to the preset experimental step data. Executed experimental time of the target sample is determined according to the currently-executed step data. The remaining experimental time data are obtained by computing a difference between the total preset experimental time and the executed experimental time, and the remaining experimental time data are taken as the experimental state data of the target sample.

In some embodiments of the present disclosure, in the case that the experimental state data indicate the in-experimental state data, total preset experimental time of the target sample may be further determined according to the preset experimental step data in order to facilitate understanding the remaining experimental time by the user. The preset experimental step data include time required by the experimental steps, and the total preset experimental time of the target sample can be obtained by summing the time required by the steps of the experiment. According to the currently-executed step data, a currently executed step may be determined, and the executed experimental time may be obtained by summing time required by steps before the currently executed step. That is, the executed experimental time of the target sample is determined according to the currently-executed step data.

After the total preset experimental time and the executed experimental time are obtained, the remaining experimental time data are obtained by computing a difference between the total preset experimental time and the executed experimental time, and the remaining experimental time data are taken as the experimental state data of the target sample.

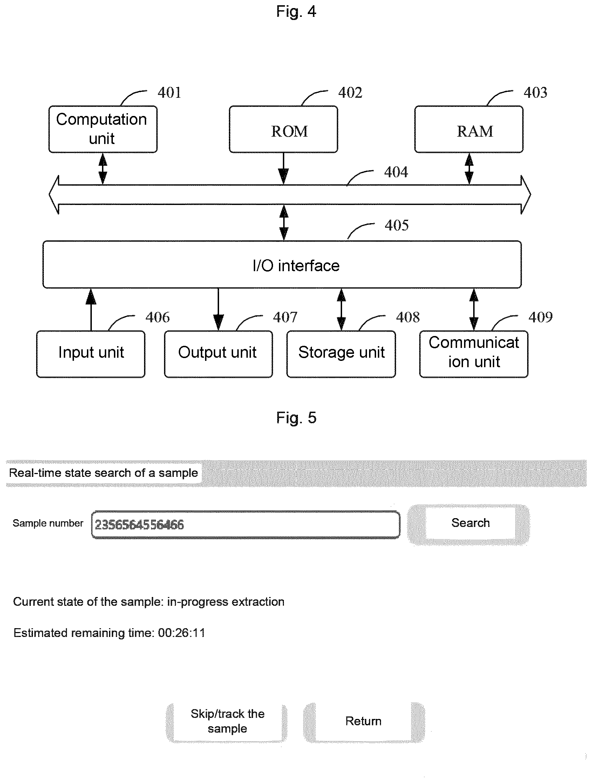

With reference to an image of a sample real-time state search interface shown in Fig. 5, a sample number "2356564556466" is configured to determine the target sample, and the user transmits the experimental state search instruction of the target sample by inputting the sample number. After the experimental state data of the target sample (shown as in-progress extraction in the figure) are determined, the page is used to display the experimental state data. The experimental state data further include the remaining experimental time data, that is, estimated remaining time.

It should be noted that in some cases, a function of skip/sample tracking may be unavailable, merely the real-time state search of the sample and the estimated remaining time are available, or even merely the real-time state search of the sample rather than the estimated remaining time is available.

In some embodiments of the present disclosure, the preset experimental step data include step execution time data, and the experimental state data indicate the in-experimental state data, the method further includes: steps to be executed are determined according to the currently-executed step data. Execution time of the steps to be executed is determined according to the preset experimental step data. The remaining experimental time data are obtained by summing the execution time of the steps to be executed, and the remaining experimental time data are taken as the experimental state data of the target sample.

In some embodiments of the present disclosure, the preset experimental step data include the time required by the experimental steps. In order to facilitate understanding the remaining experimental time by the user, in the case that the experimental state data indicate the in-experimental state data, the steps to be executed are determined according to the currently-executed step data. The execution time of the steps to be executed is determined according to the preset experimental step data. The remaining experimental time data are obtained by summing the execution time of the steps to be executed, and the remaining experimental time data are taken as the experimental state data of the target sample.

In some embodiments of the present disclosure, in a case that the experimental state data indicate the pre-experimental state data, the step that a display sub-content corresponding to the experimental state data is obtained includes: registration interface information corresponding to the currently-executed step data is obtained. The registration interface information includes sample storage position information of a biological sample experiment, and the sample storage position information includes sample rack information and sample rack position information. The step that the display sub-content and the symbol information are displayed on a second display area includes: the registration interface information is displayed on the second display area, and the sample rack information and the sample rack position information corresponding to the target sample are symbolized by using the symbol information.

In some embodiments of the present disclosure, relevant data of an interface displayed during registration of the experimental data of the biological sample experiment to which the currently-executed step data belongs are taken as the registration interface information corresponding to the currently-executed step data. In the case that the experimental state data indicate the pre-experimental state data, the registration interface information corresponding to the currently-executed step data is obtained, and the registration interface information is taken as the display sub-content. The registration interface information including data configured to be displayed in a registration interface. The registration interface is the interface displayed when the experimental data are registered before the experiment. For example, the registration interface may be an interface used in a sample analyzer to set a correspondence relationship between the sample number and a mechanical physical position where the sample is placed when the sample is loaded.

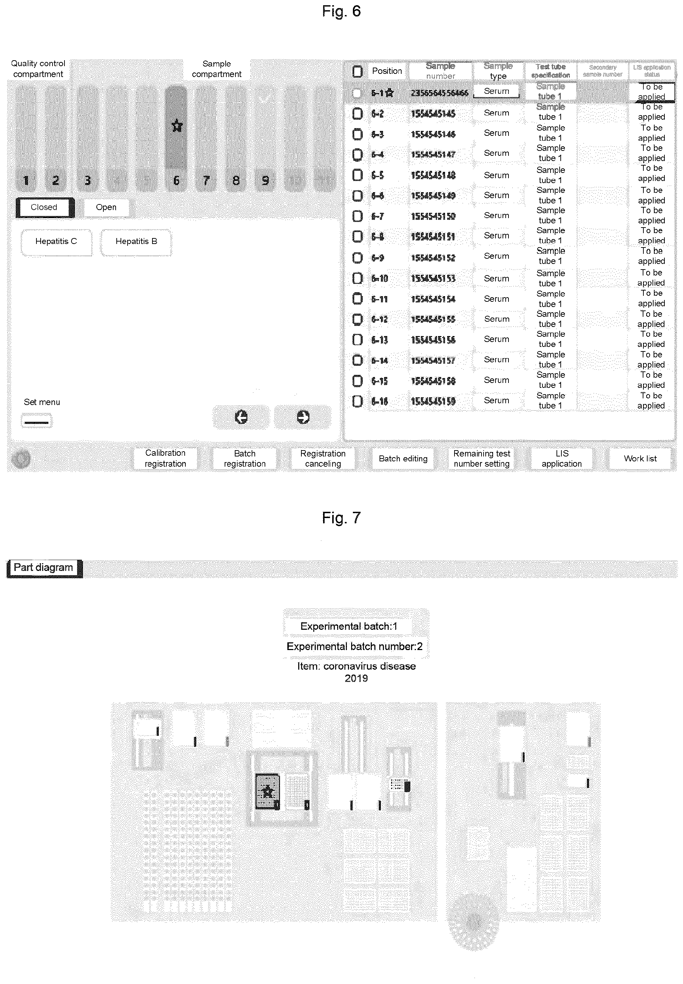

The registration interface information includes the sample storage position information of the biological sample experiment, and the sample storage position information includes the sample rack information and the sample rack position information. When the solution is implemented specifically, the registration interface information is taken as a tracking display content. With reference to an image I of a sample tracking display interface shown in Fig. 6, the sample storage position information may include quality control compartment information and sample compartment information. A quality control compartment includes a sample rack 1 and a sample rack 2. A sample compartment includes a sample rack 3 to a sample rack 11, and a star is used as symbol information to symbolize a sample rack 6, that is, the sample rack 6 is a rack where the target sample is located.

On the right in Fig. 6, the sample rack position information is displayed, and specifically includes: a position 6-1 to a position 6-16. A star is used as symbol information to symbolize a first position of the sample rack, that is 6-1. The position is a position of the sample rack where the target sample is located.

It should be noted that the registration interface information may further include data such as a sample type, a test tube specification, a secondary sample number and an application state of a laboratory information system (LIS). Data contents in the registration interface information may be determined according to actual demand, which is not specifically limited by the embodiments of the present disclosure.

The registration interface information is displayed on the second display area and the sample rack information and the sample rack position information corresponding to the target sample are symbolized by using the symbol information, such that the user can rapidly understand a current process progress of the target sample.

In some embodiments of the present disclosure, in a case that the experimental state data indicate the in-experimental state data, the step that a display sub-content corresponding to the experimental state data is obtained includes: part diagram information corresponding to the biological sample experiment is obtained. The part diagram information includes image information of a part that is configured to execute the biological sample experiment. The step that symbol information corresponding to the target sample in the display sub-content is generated includes: symbol information of image information of a part where the target sample is currently positioned in the part diagram information is generated. The step that the display sub-content and the symbol information are displayed on a second display area includes: the part diagram information is displayed on the second display area, and a part position corresponding to the target sample is symbolized by using the symbol information.

In some embodiments of the present disclosure, in the case that the experimental state data indicate the in-experimental state data, the part diagram information corresponding to the biological sample experiment is obtained. The part diagram information is taken as the display sub-content corresponding to the experimental state data. The part diagram information includes the image information of the part that is configured to execute the biological sample experiment. It should be noted that the part diagram is drawn according to an actual mechanical part structure of a sample analyzer, and some key parts (parts through which the sample flows) in the diagram are allowed to be updated by software in real time, and a symbol is added. In general, the part diagram is a mechanical part layout diagram with a local display content modifiable.

The symbol information of the image information of the part where the target sample is currently positioned in the part diagram information is generated. The part diagram information is displayed on the second display area, and the part position corresponding to the target sample is symbolized by using the symbol information. Thus, the part position where the target sample is located can be highlighted, and the user can intuitively understand a flow situation of the biological sample among machine parts.

With reference to an image II of the sample tracking display interface shown in Fig. 7. Shapes with different sizes and different gray colors indicate different parts. In this figure, a star is used as symbol information to symbolize the part position where the target sample is located. If the target sample has separated samples, a part position where each separated sample is located is symbolized with a star similarly.

It should be noted that there are black vertical lines in a lower right corner of a plurality of rectangular positions in the figure. When such a position is clicked with a mouse, experimental information, such as an experimental batch, an experimental batch number and an experimental item, is displayed. In the case of sample flow in the part, the rectangular position with a black vertical line in the lower right corner is displayed as a position circled by a dotted line box, so as to facilitate understanding the sample flow in the part by the user. In some embodiments of the present disclosure, the step that the part diagram information is displayed on the second display area further includes: in a case that a part detail display instruction is received, name information of a part corresponding to the part detail display instruction and experimental information corresponding to the target sample are displayed.

In some embodiments of the present disclosure, the detail display instruction is configured to further display detailed information in the part diagram. The detailed information may include, but is not limited to, the name information of the part and the experimental information. The experimental information includes, but is not limited to, experimental item information and/or experimental batch information. The detail display instruction may be transmitted by the user, and a specific transmission mode may be set according to actual demand. For example, in a case that the second display area is a touch screen, the user may transmit a part detail display instruction by touching a part on the screen with a finger. In a case that the second display area is a display device, the user may transmit a part detail display instruction by clicking a part in the screen with a mouse or hovering the mouse over a part in the screen for a certain period of time, and may further transmit the detail display instruction by selecting a part through voice.

One sample may have a plurality of objects to be tested during a loading test. Thus, the sample may be separated and then processed differently in different experimental consumables. In this case, different separated samples of the same sample may flow in a plurality of mechanical parts of the part diagram at the same time. Thus, when a current flow position of the sample in the part diagram is tracked, a plurality of symbols may exist. The plurality of symbols indicate flow situations of different separated samples of the sample. The plurality of symbols may be the same or not, and are configured to track respective flow situations of different separated samples. Based on that, in some embodiments of the present disclosure, in a case the target sample includes a plurality of separated samples, the step that symbol information of image information of a part where the target sample is currently positioned in the part diagram information is generated includes: symbol information of image information of parts where the separated samples are currently positioned are generated respectively. The step that a part position corresponding to the target sample is symbolized by using the symbol information includes: part positions corresponding to the separated samples are symbolized by using the symbol information of the separated samples respectively.

In some embodiments of the present disclosure, in the process of processing the biological sample, it is necessary to separate the biological sample into different components by a physical or chemical method sometimes, and each of such components are referred to as a separated sample. The plurality of separated samples may be obtained based on the target sample. Thus, in order to facilitate understanding the experimental state of the separated samples by the user, when the symbol information of the image information of the part where the target sample is currently positioned in the part diagram information is generated, the symbol information of the image information of parts where the separated samples are currently positioned may be generated respectively. The part positions corresponding to the separated samples are symbolized by using the symbol information of the separated samples respectively, thereby highlighting the flow situation of the separated samples in the parts.

In some embodiments of the present disclosure, in a case that the experimental state data indicate the post-experimental state data, the step that a display sub-content corresponding to the experimental state data is obtained includes: result interface information corresponding to the currently-executed step data is obtained. The result interface information includes sample result information of a biological sample experiment, and the sample result information includes basic sample information and to-be-tested matter result information. The step that the display sub-content and the symbol information are displayed on a second display area includes: the result interface information is displayed on the second display area, and the sample result information corresponding to the target sample is symbolized by using the symbol information.

In some embodiments of the present disclosure, relevant data of an interface displayed during registration of an experimental result of the biological sample experiment to which the currently-executed step data belongs are taken as the result interface information corresponding to the currently-executed step data. In the case that the experimental state data indicate the post-experimental state data, the result interface information corresponding to the currently-executed step data is obtained, and the result interface information is taken as the display sub-content. The result interface information including data configured to be displayed in a result interface. The result interface is the interface displayed when the experimental result is registered after the experiment. For example, the result interface may be an interface used in a sample analyzer to display a sample test result. In this interface, the user can view some/all test result information of a tested sample.

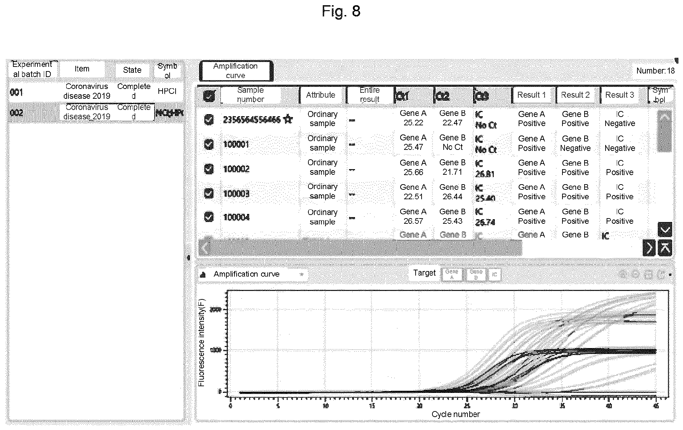

The result interface information includes the sample result information of the biological sample experiment, and the sample result information includes the basic sample information and the to-be-tested matter result information. When the solution is specifically implemented, the result interface information is taken as the tracking display content. With reference to an image III of the sample tracking display interface shown in Fig. 8, the basic sample information shown in the figure includes an experimental item, an experimental state, an experimental symbol and/or an experimental batch identity (ID), etc. The to-be-tested matter result information shown in the figure includes a sample number, an attribute, an entire result, an object to be tested and a corresponding test result of the object to be tested.

It should be noted that the result interface information may further include data such as a reaction curve and/or temperature curve. Data contents in the result interface information may be determined according to actual demand, which is not specifically limited by the embodiments of the present disclosure.

The result interface information is displayed on the second display area and the sample result information corresponding to the target sample is symbolized by using the symbol information, such that the user can timely understand a process progress of the target sample after the experiment. For example, in Fig. 8, the experimental result of the sample number "2356564556466" is symbolized with a star as the symbol information.

The implementation of this method will be described with a specific embodiment.

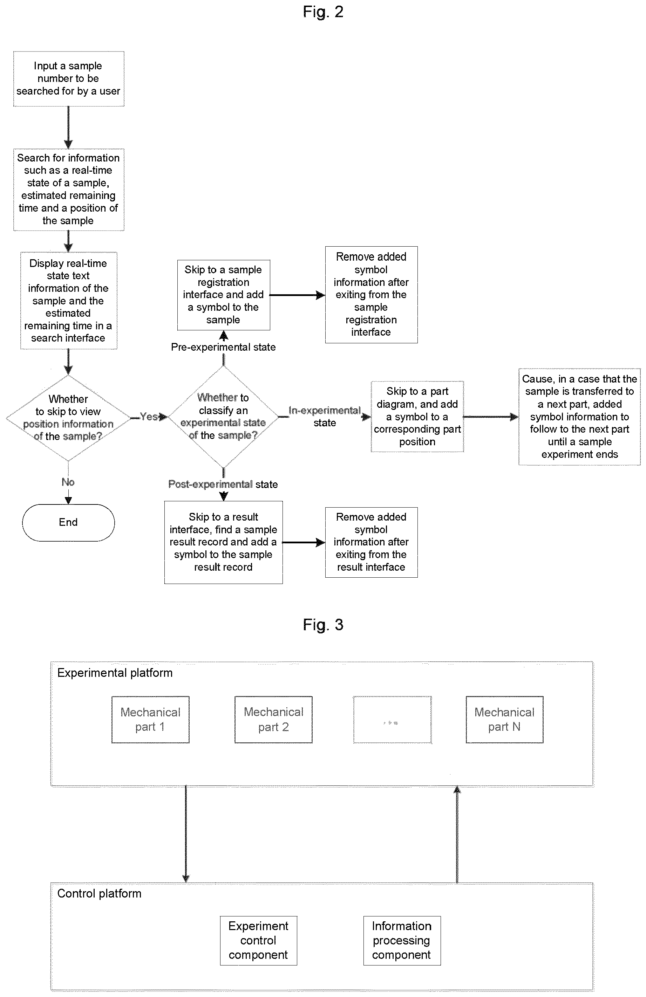

The method may be implemented based on a system for analyzing a biological sample. In some embodiments of the present disclosure, as shown in an entire architecture diagram of a system for analyzing a biological sample in Fig. 3, the system may include an experimental platform and a control platform. The experimental platform includes one or more mechanical parts, and these mechanical parts are configured to complete specific experimental actions. The plurality of parts cooperate with one another to complete a sample test experiment, and a sample or a sample extract flow through these mechanical parts. The control platform includes an experiment control component and an information processing component. The experiment control component controls movement of various mechanical parts in the experimental platform by generating a control instruction and transmitting the instruction to the experimental platform and completes the experimental process. The information processing component is configured to manage sample registration information and sample result information in the instrument and draw/display the key part diagram corresponding to the mechanical structure of the instrument. The key part diagram corresponding to the mechanical structure of the instrument is simply referred to as the "part diagram" for the convenience of description.

The experimental platform and the control platform may communicate with each other through various existing data communication methods, including but not limited to network connections such as a local area network (LAN), a virtual private network (VPN), intranet, Internet, a serial port connection, a universal serial bus (USB) connection and a radio network connection.

Real-time state search and tracking of the sample may be implemented based on the sample registration information, the sample result information and the part diagram in the information processing component. A specific method is as follows:
1) The sample real-time state search interface is set, the user inputs the sample number to be searched for, and the search operation is executed.
2) According to the sample number input by the user, the sample registration information, the sample result information and the part diagram are retrieved. The real-time state of the sample, the estimated remaining time and the current mechanical position of the sample are obtained.
3) Real-time state text information (such as registered, applied, in-progress sample adding, in-progress reagent adding, in-progress reacting, in-progress testing and result output completion) of a current sample and the estimated remaining time are displayed in the sample real-time state search interface. A skip function is further provided to allow the user to skip directly to the sample registration interface/the result interface/a part diagram interface to view the current state and position information of the sample.
4) A specific interface to be skipped to needs to be determined according to the current state of the sample. Generally, before starting to process the sample, it belongs to pre-experimental, and should skip to the sample registration interface. From the beginning of sample processing and before result is obtained, it belongs to in-experimental, and should skip to the part diagram. After the experiment is end, it belongs to post-experimental, and should skip to the result interface. Thus, the real-time state of the sample may be divided into the pre-experimental state, the in-experimental state and the post-experimental state. Each class of state may include one or more subdivided states. For example, the pre-experimental state may be divided into registered and applied. The in-experimental state may be divided into the in-progress uncapping, the in-progress sample adding, the in-progress reagent adding, the in-progress reacting, the in-progress testing, etc. The post-experimental state may be divided into the result output completion, audit completion. etc.
5) After skip to a specific page, a position/a record of a retrieved sample is highlighted by adding a symbol. Specifically, if the sample is in the in-experimental state, a current flow position of the retrieved sample is highlighted by adding the symbol to a position of a relevant part of the part diagram. In addition, the symbol information flows and is displayed in the parts following the sample and along with the progress of the experiment until the end of the test experiment of the sample. A specific flowchart of searching for and tracking a real-time state of a sample is shown in Fig. 2.

A specific implementation of the present disclosure will be described with an automatic nucleic acid test analyzer as an example. The sample real-time state search interface is shown in Fig. 5. The sample real-time state search interface includes: a sample number input box (as shown in the figure: a sample number input box), a search button (as shown in the figure, for searching for a sample with the sample number of 2356564556466), current state text information of the sample (as shown in the figure, the current state of the sample is "in-progress extraction"), and the estimated remaining time of the sample (as shown in the figure, the estimated remaining time of the sample is "00:26:11"), a skip/sample tracking button (configured for interface skip to further search for a sample rack/part position/result record of the sample), and a return button (configured to terminate the sample state search process).

When the sample state is the "pre-experimental state", the skip/sample tracking button is clicked, and software automatically navigates to the sample registration interface and adds a symbol to a corresponding sample position. As shown in Fig. 6, a red star is used to symbolize the sample rack and the position of the sample.

When the sample state is the "in-experimental state", the skip/sample tracking button is clicked, and software automatically navigate to the part diagram interface and adds a symbol to a current flow part of the sample. As shown in Fig. 7, a red star is used to symbolize a mechanical part of the sample, and the symbol is automatically displayed in a next mechanical part along with the sample.

When the sample state is the "post-experimental state", the skip/sample tracking button is clicked, and software automatically navigates to the result interface and automatically finds the result record of the sample and adds a symbol to the result record. As shown in Fig. 8, a red star is used to symbolize the result record of the sample.

Based on the method according to the embodiments of the present disclosure, the embodiments of the present disclosure further provide an apparatus for displaying an experimental state of a biological sample. The apparatus includes: an obtainment component configured to obtain, in a case that an experimental state search instruction of a target sample is received, currently-executed step data of the target sample; a data component configured to determine experimental state data of the target sample according to preset experimental step data and the currently-executed step data; a first display component configured to display the experimental state data on a first display area; and a second display component configured to, in a case that a tracking display instruction is received, obtain a display sub-content corresponding to the experimental state data, generate symbol information corresponding to the target sample in the display sub-content, and display the display sub-content and the symbol information on a second display area.

In some embodiments of the present disclosure, in a case that the preset experimental step data include a correspondence relationship between the currently-executed step data and the experimental state data; the step that experimental state data of the target sample are determined according to preset experimental step data and the currently-executed step data includes: according to the correspondence relationship, experimental state data corresponding to the currently-executed step data are determined. The experimental state data include pre-experimental state data, in-experimental state data and post-experimental state data, a pre-experimental state includes at least one or more of the following states: registered and applied, an in-experimental state includes at least one or more of the following states: in-progress uncapping, in-progress sample adding, in-progress reagent adding, in-progress reacting and in-progress testing, and a post-experimental state includes at least one or more of the following states: result output completion and audit completion. The experimental state data corresponding to the currently-executed step data are taken as the experimental state data of the target sample.

In some embodiments of the present disclosure, in a case that the experimental state data indicate the in-experimental state data, the method further includes: total preset experimental time of the target sample is determined according to the preset experimental step data. Executed experimental time of the target sample is determined according to the currently-executed step data. The remaining experimental time data are obtained by computing a difference between the total preset experimental time and the executed experimental time, and the remaining experimental time data are taken as the experimental state data of the target sample.

In some embodiments of the present disclosure, in cases that the preset experimental step data include step execution time data, and the experimental state data indicate the in-experimental state data, the method further includes: steps to be executed are determined according to the currently-executed step data. Execution time of the steps to be executed is determined according to the preset experimental step data. The remaining experimental time data are obtained by summing the execution time of the steps to be executed, and the remaining experimental time data are taken as the experimental state data of the target sample.

In some embodiments of the present disclosure, in a case that the experimental state data indicate the pre-experimental state data, the step that a display sub-content corresponding to the experimental state data is obtained includes: registration interface information corresponding to the currently-executed step data is obtained. The registration interface information includes sample storage position information of a biological sample experiment, and the sample storage position information includes, but is not limited to, sample rack information and sample rack position information. The step that the display sub-content and the symbol information are displayed on a second display area includes: the registration interface information is displayed on the second display area, and the sample rack information and the sample rack position information corresponding to the target sample are symbolized by using the symbol information.

In some embodiments of the present disclosure, in a case that the experimental state data indicate the in-experimental state data, the step that a display sub-content corresponding to the experimental state data is obtained includes: part diagram information corresponding to the biological sample experiment is obtained. The part diagram information includes image information of a part that is configured to execute the biological sample experiment. The step that symbol information corresponding to the target sample in the display sub-content is generated includes: symbol information of image information of a part where the target sample is currently positioned in the part diagram information is generated. The step that the display sub-content and the symbol information are displayed on a second display area includes: the part diagram information is displayed on the second display area, and a part position corresponding to the target sample is symbolized by using the symbol information.

In some embodiments of the present disclosure, the step that the part diagram information is displayed on the second display area further includes: in a case that a part detail display instruction is received, name information of a part corresponding to the part detail display instruction and experimental information corresponding to the target sample are displayed.

In some embodiments of the present disclosure, the target sample includes a plurality of separated samples, the step that symbol information of image information of a part where the target sample is currently positioned in the part diagram information is generated includes: symbol information of image information of parts where the separated samples are currently positioned are generated respectively. The step that a part position corresponding to the target sample is symbolized by using the symbol information includes: part positions corresponding to the separated samples are symbolized by using the symbol information of the separated samples respectively.

In some embodiments of the present disclosure, in a case that the experimental state data indicate the post-experimental state data, the step that a display sub-content corresponding to the experimental state data is obtained includes: result interface information corresponding to the currently-executed step data is obtained. The result interface information includes sample result information of a biological sample experiment, and the sample result information includes basic sample information and to-be-tested matter result information. The step that the display sub-content and the symbol information are displayed on a second display area includes: the result interface information is displayed on the second display area, and the sample result information corresponding to the target sample is symbolized by using the symbol information.

In some embodiments of the present disclosure, the step that symbol information corresponding to the target sample in the display sub-content is generated includes: one or more combinations of data corresponding to the target sample are generated as follows: shape data, color data and text data.

Based on the method according to the embodiments of the present disclosure, the embodiments of the present disclosure further provide a system for analyzing a biological sample is further provided. The system includes: an experimental platform and a control platform; where the experimental platform is in communication connection to the control platform, the control platform includes an experiment control component and an information processing component, and the information processing component is configured to execute the method described above.

In some embodiments of the present disclosure, the experiment control component of the control platform may transmit experiment execution step data to the experimental platform. The information processing component of the control platform also records the experiment execution step data. The experimental platform may include a variety of instruments for executing the biological sample experiment. The experimental platform receives the experiment execution step data. The received experiment execution step data may be configured to instruct the instrument that is configured to execute the biological sample experiment in the experimental platform to complete a specific mechanical motion. After the mechanical motion is completed, the experimental platform feeds back an execution situation of a corresponding experimental step to the experiment control component of the control platform. The information processing component obtains the currently-executed step data from the experiment control component. The currently-executed step data are configured to describe an execution state of the current step.

The experiment control component repeats the process, continues to transmit next experiment execution step data, and pushes the experiment of the sample forwards.

Based on the method according to the embodiments of the present disclosure, the embodiments of the present disclosure further provide a system for analyzing a biological sample is further provided. The system includes: a test apparatus configured to obtain a sample test result by testing a sample liquid to be tested; a display; and a controller; where in a case that an experimental state search instruction of a target sample is detected, the controller is configured to control the display to display a first display area, and the first display area is configured to display experimental state data of the target sample; and on the basis of detecting a tracking display instruction of the target sample, the controller is configured to obtain a display sub-content corresponding to the experimental state data, generate symbol information corresponding to the target sample in the display sub-content, and control the display to display a second display area, and the second display area is configured to display the display sub-content and the symbol information corresponding to the experimental state data of the target sample.

In some embodiments of the present disclosure, the test apparatus may be a biological sample analyzer. The biological sample analyzer refers to an instrument for testing and analyzing a sample to be tested to obtain a test result of the sample to be tested. The biological sample analyzer specifically may be a biological sample analyzer such as a nucleic acid test analyzer, a biochemical analyzer and an immunoassay analyzer. The biological sample analyzer includes at least a consumable loading component, a sample filling component, a reagent filling component, a reaction component and a measurement component.

The system for analyzing a biological sample also includes a display apparatus, that is, a display. The display apparatus is configured to provide a human-machine interaction interface. The display apparatus may be arranged in the same instrument as a part of the biological sample analyzer, or may be arranged outside the instrument where the part of the biological sample analyzer is located, and keep a communication connection to the part.

The interaction interface provided by the display apparatus may display parameter values of test indexes, a device state, a consumables state, a sample test state, etc. The user may input a related operation into the human-computer interaction interface, to transmit an instruction to the system for analyzing a biological sample. The related operation may be a mouse operation, a touch operation or a keyboard input.

In some embodiments of the present disclosure, in a case that the preset experimental step data include a correspondence relationship between the currently-executed step data and the experimental state data; the step that experimental state data of the target sample are determined according to preset experimental step data and the currently-executed step data includes: according to the correspondence relationship, experimental state data corresponding to the currently-executed step data are determined. The experimental state data include pre-experimental state data, in-experimental state data and post-experimental state data, a pre-experimental state includes at least one or more of the following states: registered and applied, an in-experimental state includes at least one or more of the following states: in-progress uncapping, in-progress sample adding, in-progress reagent adding, in-progress reacting and in-progress testing, and a post-experimental state includes at least one or more of the following states: result output completion and audit completion. The experimental state data corresponding to the currently-executed step data are taken as the experimental state data of the target sample.

In some embodiments of the present disclosure, in a case that the experimental state data indicate the in-experimental state data, the method further includes: total preset experimental time of the target sample is determined according to the preset experimental step data. Executed experimental time of the target sample is determined according to the currently-executed step data. The remaining experimental time data are obtained by computing a difference between the total preset experimental time and the executed experimental time, and the remaining experimental time data are taken as the experimental state data of the target sample.

In some embodiments of the present disclosure, in cases that the preset experimental step data include step execution time data, and the experimental state data indicate the in-experimental state data, the method further includes: steps to be executed are determined according to the currently-executed step data. Execution time of the steps to be executed is determined according to the preset experimental step data. The remaining experimental time data are obtained by summing the execution time of the steps to be executed, and the remaining experimental time data are taken as the experimental state data of the target sample.

In some embodiments of the present disclosure, in a case that the experimental state data indicate the pre-experimental state data, the step that a display sub-content corresponding to the experimental state data is obtained includes: registration interface information corresponding to the currently-executed step data is obtained. The registration interface information includes sample storage position information of a biological sample experiment, and the sample storage position information includes, but is not limited to, sample rack information and sample rack position information. The step that the display sub-content and the symbol information are displayed on a second display area includes: the registration interface information is displayed on the second display area, and the sample rack information and the sample rack position information corresponding to the target sample are symbolized by using the symbol information.

In some embodiments of the present disclosure, in a case that the experimental state data indicate the in-experimental state data, the step that a display sub-content corresponding to the experimental state data is obtained includes: part diagram information corresponding to the biological sample experiment is obtained. The part diagram information includes image information of a part that is configured to execute the biological sample experiment. The step that symbol information corresponding to the target sample in the display sub-content is generated includes: symbol information of image information of a part where the target sample is currently positioned in the part diagram information is generated. The step that the display sub-content and the symbol information are displayed on a second display area includes: the part diagram information is displayed on the second display area, and a part position corresponding to the target sample is symbolized by using the symbol information.

In some embodiments of the present disclosure, the step that the part diagram information is displayed on the second display area further includes: in a case that a part detail display instruction is received, name information of a part corresponding to the part detail display instruction and experimental information corresponding to the target sample are displayed.

In some embodiments of the present disclosure, in a case the target sample includes a plurality of separated samples, the step that symbol information of image information of a part where the target sample is currently positioned in the part diagram information is generated includes: symbol information of image information of parts where the separated samples are currently positioned are generated respectively. The step that a part position corresponding to the target sample is symbolized by using the symbol information includes: part positions corresponding to the separated samples are symbolized by using the symbol information of the separated samples respectively.

In some embodiments of the present disclosure, in a case that the experimental state data indicate the post-experimental state data, the step that a display sub-content corresponding to the experimental state data is obtained includes: result interface information corresponding to the currently-executed step data is obtained. The result interface information includes sample result information of a biological sample experiment, and the sample result information includes basic sample information and to-be-tested matter result information. The step that the display sub-content and the symbol information are displayed on a second display area includes: the result interface information is displayed on the second display area, and the sample result information corresponding to the target sample is symbolized by using the symbol information.

In some embodiments of the present disclosure, the step that symbol information corresponding to the target sample in the display sub-content is generated includes: one or more combinations of data corresponding to the target sample are generated as follows: shape data, color data and text data.

The embodiments of the present disclosure further provide an electronic device. The electronic device include: at least one processor and a memory; and a memory in communication connection to the at least one processor. The memory stores a computer program that is executable by the at least one processor, and the computer program is configured to make the electronic device execute the method of the embodiments of the present disclosure when executed by the at least one processor.

Embodiments of the present disclosure further provide a non-transitory machine-readable medium storing a computer program. The computer program is configured to make a computer execute the method of the embodiments of the present disclosure when executed by a processor of the computer.

Embodiments of the present disclosure further provide a computer program product. The computer program product includes a computer program. The computer program is configured to make a computer execute the method of the embodiments of the present disclosure when executed by a processor of the computer.

With reference to Fig. 4, a structural block diagram of an electronic device that may serve as a server or a client of the embodiments of the present disclosure is now described, which is an instance of a hardware device that may be applied to various aspects of the present disclosure. The electronic device is intended to indicate various forms of digital electronic computer devices, such as a laptop computer, a desktop computer, a workstation, a personal digital assistant, a server, a blade server, a mainframe computer, and other suitable computers. The electronic device may also indicate various forms of mobile apparatuses, such as personal digital processing, a cellular phone, a smart phone, a wearable device and other similar computing apparatuses. The parts shown herein, their connections and relationships, and their functions are merely illustrative, and are not intended to limit implementation of the present disclosure described and/or claimed herein.

As shown in Fig. 4, the electronic device includes a computation unit 401. The computation unit may execute various appropriate actions and processing according to a computer program stored in a read-only memory (ROM) 402 or a computer program loaded from a storage unit 408 into a random access memory (RAM) 403. The RAM 403 may further store various programs and data required for the operation by the electronic device. The computation unit 401, the ROM 402, and the RAM 403 are connected to one another through a bus 404. An input/output (I/O) interface 405 is also connected to the bus 404.

A plurality of parts including an input unit 406, an output unit 407, the storage unit 408, and a communication unit 409 of the electronic device are connected to the I/O interface 405. The input unit 406 may be any type of device capable of inputting information into the electronic device. The input unit 406 may receive input digit or character information, and generate a key signal input related to user settings and/or function control of the electronic device. The output unit 407 may be any type of device capable of displaying information, and may include, but is not limited to, a display, a speaker, a video/audio output terminal, a vibrator, and/or a printer. The storage unit 408 may include, but is not limited to, a magnetic disk and an optical disc. The communication unit 409 allows the electronic device to exchange information/data with other devices via a computer network such as the computer Internet and/or various telecommunications networks, and may include, but is not limited to, a modem, a network interface card, an infrared communication device, a radio communication transceiver and/or a chipset, for example, a Bluetooth device, a Wi-Fi device, a WiMax device, a cellular communication device and/or the like.

The computation unit 401 may be various general-purpose and/or special-purpose processing components with processing and computing capabilities. Some instances of the computation unit 401 include, but are not limited to, a central processing unit (CPU), a graphics processing unit (GPU), various dedicated artificial intelligence (Al) computing chips, various computation units that run machine learning model algorithms, a digital signal processor (DSP), and any appropriate processor, controller, microcontroller, etc. The computation unit 401 executes the methods and processing described above. For example, in some embodiments of the present disclosure, the method embodiments of the present disclosure may be implemented as a computer program, which is tangibly contained in a machine-readable medium, such as the storage unit 408. In some examples, some or all of the computer programs may be loaded and/or installed into the electronic device via the ROM 402 and/or the communication unit 409. In some embodiments of the present disclosure, the computation unit 401 may be configured, by any other suitable means (for example, by means of firmware), to execute the method described above.

The computer program configured to implement the method of the embodiments of the present disclosure may be written in any combination of one or more programming languages. These computer programs may be provided for a processor or controller of a general-purpose computer, a special-purpose computer or other programmable data processing apparatus, such that the computer programs cause the function/operation specified in the flowcharts and/or block diagrams to be implemented when executed by the processor or the controller. The computer program may be completely executed on a machine, partially executed on a machine, partially executed on a machine and partially executed on a remote machine as a separate software package or completely executed on a remote machine or a server.

In the context of the embodiments of the present disclosure, a machine-readable medium may be a tangible medium, and may include or store a program that is used by or in combination with an instruction execution system, apparatus or device. The machine-readable medium may be a machine-readable signal medium or a machine-readable storage medium. The machine-readable signal medium may include, but is not limited to, an electric, magnetic, optical, electromagnetic, infrared, or semiconductor system, apparatus or device, or their any suitable combination. More specific instances of the machine-readable storage medium may include an electrical connection based on one or more wires, a portable computer disk, a hard disk, a random access memory (RAM), a read-only memory (ROM), an erasable programmable read-only memory (EPROM) or a flash memory, an optical fiber, a portable compact disk read-only memory (CD-ROM), an optical storage device, a magnetic storage device, or their any suitable combination.

It should be noted that the terms "comprise", "include" and their variations used in the embodiments of the present disclosure are open-ended, that is, "comprise but not limited to" and "include but not limited to". The term "based on" indicates "at least partially based on". The term "an embodiment" indicates "at least one embodiment". The term "another embodiment" indicates "at least another one embodiment". The term "some embodiments" indicates "at least some embodiments". Modifications with "a", "an" and "a plurality of" mentioned in the embodiments of the present disclosure are illustrative rather than limitative, and should be understood by those skilled in the art as "one or more" unless otherwise indicated definitely in the context.

User information (including but not limited to user device information and user personal information), and data (including but not limited to data for analysis, data stored and data displayed) that are involved in the embodiments of the present disclosure are information and data authorized by a user or fully authorized by all parties. In addition, the collection, use and processing of relevant data should comply with relevant laws, regulations and standards in relevant countries and regions, and a corresponding operation entry is provided for the user to confirm or reject authorization.

The steps described in a method embodiment provided by the embodiments of the present disclosure can be executed in different orders and/or in parallel. Further, the method embodiment can include an additional step and/or omit a shown step, which does not limit the protection scope of the present disclosure.

The term "embodiment" in the description indicates that a specific feature, structure or characteristic described in conjunction with the embodiments can be included in at least one embodiment of the present disclosure. The phrase in various places in the description does not necessarily indicate the same embodiment, nor is it independent or alternative due to mutual exclusion with other embodiments. The embodiments in the description are described in a related manner, mutual reference can be made to the same or similar parts of the embodiments. In particular, the embodiments of the apparatus, the device and the system are basically similar to the method embodiment, and are described relatively simply as a result, and reference can be made to description of the method embodiment for relevant contents.

The embodiments described above are merely several types of embodiments of the present disclosure, and are specifically described in detail, but should not be construed as limitation to the protection scope of the patent accordingly. It should be noted that those skilled in the art can make several variations and improvements without departure from concepts of the present disclosure, and these variations and improvements should fall within the protection scope of the present disclosure. Hence, the protection scope of the present disclosure should be subject to the appended claims.

## Claims

1. A method for displaying an experimental state of a biological sample, comprising:
in a case that an experimental state search instruction of a target sample is received, obtaining currently-executed step data of the target sample;
determining experimental state data of the target sample according to preset experimental step data and the currently-executed step data;
displaying the experimental state data on a first display area; and
in a case that a tracking display instruction is received, obtaining a display sub-content corresponding to the experimental state data, generating symbol information corresponding to the target sample in the display sub-content, and displaying the display sub-content and the symbol information on a second display area.

2. The method as claimed in claim 1, wherein the preset experimental step data comprise a correspondence relationship between the currently-executed step data and the experimental state data; the determining experimental state data of the target sample according to preset experimental step data and the currently-executed step data comprises:
determining, according to the correspondence relationship, experimental state data corresponding to the currently-executed step data; wherein the experimental state data comprise pre-experimental state data, in-experimental state data and post-experimental state data, a pre-experimental state comprises at least one or more of the following states: registered and applied, an in-experimental state comprises at least one or more of the following states: in-progress uncapping, in-progress sample adding, in-progress reagent adding, in-progress reacting and in-progress testing, and a post-experimental state comprises at least one or more of the following states: result output completion and audit completion; and
taking the experimental state data corresponding to the currently-executed step data as the experimental state data of the target sample.

3. The method as claimed in claim 2, wherein the experimental state data indicate the in-experimental state data, the method further comprises:
determining total preset experimental time of the target sample according to the preset experimental step data;
determining executed experimental time of the target sample according to the currently-executed step data; and
obtaining the remaining experimental time data by computing a difference between the total preset experimental time and the executed experimental time, and taking the remaining experimental time data as the experimental state data of the target sample.

4. The method as claimed in claim 2, wherein the preset experimental step data comprise step execution time data, and the experimental state data indicate the in-experimental state data, the method further comprises:
determining steps to be executed according to the currently-executed step data;
determining execution time of the steps to be executed according to the preset experimental step data; and
obtaining the remaining experimental time data by summing the execution time of the steps to be executed, and taking the remaining experimental time data as the experimental state data of the target sample.

5. The method as claimed in claim 2, wherein the experimental state data indicate the pre-experimental state data,
the obtaining the display sub-content corresponding to the experimental state data comprises: obtaining registration interface information corresponding to the currently-executed step data; wherein the registration interface information comprises sample storage position information of a biological sample experiment, and the sample storage position information comprises sample rack information and sample rack position information; and
the displaying the display sub-content and the symbol information on the second display area comprises: displaying the registration interface information on the second display area, and symbolizing the sample rack information and the sample rack position information corresponding to the target sample by using the symbol information.

6. The method as claimed in claim 2, wherein the experimental state data indicate the in-experimental state data,
the obtaining the display sub-content corresponding to the experimental state data comprises: obtaining part diagram information corresponding to a biological sample experiment; wherein the part diagram information comprises image information of a part that is configured to execute the biological sample experiment;
the generating symbol information corresponding to the target sample in the display sub-content comprises: generating symbol information of image information of a part where the target sample is currently positioned in the part diagram information; and
the displaying the display sub-content and the symbol information on the second display area comprises: displaying the part diagram information on the second display area, and symbolizing a part position corresponding to the target sample by using the symbol information.

7. The method as claimed in claim 6, wherein the displaying the part diagram information on the second display area further comprises:
in a case that a part detail display instruction is received, displaying name information of a part corresponding to the part detail display instruction and experimental information corresponding to the target sample.

8. The method as claimed in claim 6, wherein the target sample comprises a plurality of separated samples,
the generating symbol information of image information of the part where the target sample is currently positioned in the part diagram information comprises: generating symbol information of image information of parts where the separated samples are currently positioned respectively; and
the symbolizing the part position corresponding to the target sample by using the symbol information comprises: symbolizing part positions corresponding to the separated samples by using the symbol information of the separated samples respectively.

9. The method as claimed in claim 2, wherein the experimental state data indicate the post-experimental state data,
the obtaining the display sub-content corresponding to the experimental state data comprises: obtaining result interface information corresponding to the currently-executed step data; wherein the result interface information comprises sample result information of a biological sample experiment, and the sample result information comprises basic sample information and to-be-tested matter result information; and
the displaying the display sub-content and the symbol information on the second display area comprises: displaying the result interface information on the second display area, and symbolizing the sample result information corresponding to the target sample by using the symbol information.

10. The method as claimed in claim 1, wherein the generating symbol information corresponding to the target sample in the display sub-content comprises:
generating one or more combinations of data corresponding to the target sample as follows: shape data, color data and text data.

11. A system for analyzing a biological sample, comprising:
a test apparatus configured to obtain a sample test result by testing a sample liquid to be tested;
a display; and
a controller; wherein
in a case that an experimental state search instruction of a target sample is detected, the controller is configured to control the display to display a first display area, and the first display area is configured to display experimental state data of the target sample; and
on the basis of detecting a tracking display instruction of the target sample, the controller is configured to obtain a display sub-content corresponding to the experimental state data, generate symbol information corresponding to the target sample in the display sub-content, and control the display to display a second display area, and the second display area is configured to display the display sub-content and the symbol information corresponding to the experimental state data of the target sample.

12. The system as claimed in claim 11, wherein the controller is configured to:
in a case that the experimental state search instruction of the target sample is received, obtain currently-executed step data of the target sample;
determine experimental state data of the target sample according to preset experimental step data and the currently-executed step data.

13. The system as claimed in claim 12, wherein the preset experimental step data comprise a correspondence relationship between the currently-executed step data and the experimental state data; the controller is configured to:
determine, according to the correspondence relationship, experimental state data corresponding to the currently-executed step data; wherein the experimental state data comprise pre-experimental state data, in-experimental state data and post-experimental state data, a pre-experimental state comprises at least one or more of the following states: registered and applied, an in-experimental state comprises at least one or more of the following states: in-progress uncapping, in-progress sample adding, in-progress reagent adding, in-progress reacting and in-progress testing, and a post-experimental state comprises at least one or more of the following states: result output completion and audit completion; and
take the experimental state data corresponding to the currently-executed step data as the experimental state data of the target sample.

14. The system as claimed in claim 13, wherein the experimental state data indicate the in-experimental state data, the controller is configured to:
determine total preset experimental time of the target sample according to the preset experimental step data;
determine executed experimental time of the target sample according to the currently-executed step data; and
obtain the remaining experimental time data by computing a difference between the total preset experimental time and the executed experimental time, and take the remaining experimental time data as the experimental state data of the target sample.

15. The system as claimed in claim 13, wherein the preset experimental step data comprise step execution time data, and the experimental state data indicate the in-experimental state data, the controller is configured to:
determine steps to be executed according to the currently-executed step data;
determine execution time of the steps to be executed according to the preset experimental step data; and
obtain the remaining experimental time data by summing the execution time of the steps to be executed, and take the remaining experimental time data as the experimental state data of the target sample.
